# EUROPEAN PATENT SPECIFICATION

(11) **EP 1 559 443 B1**
(45) Date of publication and mention of the grant of the patent: **10.12.2008**
(21) Application number: 04250517.2
(22) Date of filing: 30.01.2004
(51) Int. Cl.: A61M 5/315

(54) **Dosing spacer for a syringe**
Abstandshalter als Dosierhilfe für eine Spritze
Entretoise servant de dosage pour une seringue

(43) Date of publication of application: 03.08.2005
(73) Proprietor: ARES TRADING S.A., 1170 Aubonne (CH)
(72) Inventor: Gueritault, Thomas, 1008 Prilly (CH)
(74) Representative: Micheli & Cie SA

(56) References cited:
- EP-A- 0 493 639
- DE-A- 10 163 328
- US-A- 4 153 056
- US-A- 5 289 919
- US-A- 5 975 355
- US-B1- 6 368 305

## Description

The present invention relates to a device for use with a syringe which may be used to adjust the amount of fluid delivered from a syringe for injection.

Syringes for injection of various liquid medications into humans and other animals are well known in the art. In a traditional arrangement, syringes are provided with an empty fluid chamber, into which medication is drawn prior to injection. Typically, the dose of medication injected using a syringe is determined by the volume of the fluid chamber, i.e. the volume of the fluid chamber when the piston rod is drawn back to its maximum extent. If a smaller dose or volume of medication than the maximum volume of the fluid chamber is to be injected, a user either draws the piston rod back only partially until the required volume of medication is present in the fluid chamber, or fully fills the fluid chamber and' then partially depresses the piston rod until only the required volume of medication remains in the fluid chamber. A measure of the volume of fluid present in the fluid chamber could be provided by a scale on the side of the fluid chamber, which would normally be made of a transparent material such as glass.

The traditional arrangement described above requires a user to accurately measure the amount of fluid being drawn into the fluid chamber of a syringe. Mistakes in this measurement could lead to an incorrect dose of medication being administered which could have very serious effects. Consequently, various ways of more accurately controlling a dose of medication administered from a syringe have been developed.

One method of controlling the dosage of medication administered from a syringe comprises using a spacer element of a predetermined length which is arranged around a piston rod of a syringe. In use, the spacer comes into abutment against a finger pad at the top of the piston rod when the piston has been partially depressed and so stops the piston from being depressed any further. Consequently, only a known portion of the total volume of medication held in the fluid chamber is administered, and the amount of medication administered depends on the length of the spacer element.

Examples of known spacer elements are given in US 5,975,355A and US 2,855,928A. Both of these documents disclose the use of solid spacer blocks having a groove provided in one side edge thereof, in which a piston rod of a syringe is received in use. The spacer blocks of US 5,975,355A are reusable and can be placed on to the piston rod of a syringe and removed as many times as required. However, as the piston rod is inserted into an open sided groove in the block, the user must hold the spacer block onto the piston rod during injection of a medicament. This makes the spacer block relatively difficult to use, especially by a patient injecting themselves or by the elderly. Further, if the spacer block slips during use, the wrong dose of medicament can be injected leading to potentially serious effects on a patient.

US 5,833,669 discloses a disposable spacer collar which is provided on a single use pre-filled syringe. In one embodiment, the spacer is provided with a thin portion which means that it can be torn open to remove it from the syringe. However, the spacer collar cannot be provided separately from a syringe with which it is to be used and is also not reusable.

Document US-A-4 153 056 discloses a variable dose syringe having a removable length adjusting member or "nut" which is threadably engaged on the plunger. In one embodiment, the removable length-adjusting member can be a one-piece hinged element having two adjacent open end portions which can be realeasably secured to each other to form a complete annular nut. When it is desired to place the nut on the stem of the plunger, the end portions of the nut are opened, the ends are spread apart, and the plunger is inserted therebetween.

Document US-B-6 368 305 B1 discloses a syringe plunger immobilizing device including first and second hollow members, a means for connecting the first and second hollow members to one another, and a means for holding the first and second hollow members in contact with one another. In this fashion the syringe may be preloaded with a medicament and then safely transported without loss of the medicament within the syringe until the syringe is used.

From a first aspect, the present invention provides a device for use with a syringe, the device comprising:
a longitudinal axis;
a hollow body extending about the longitudinal axis;
a first opening provided at a first end of the hollow body;
a second opening provided at a second end of the hollow body, the second end being opposite to and longitudinally spaced from the first end; and
a slit extending over the length of the hollow body,
wherein the hollow body is adapted to be opened about the slit such that in use the hollow body may be placed around and closed over the piston rod of a syringe,
the device being characterised in that the hollow body is movably mountable on the piston rod such that in use, by pushing the piston rod, a flange of the piston rod may be brought into abutment with the first end of the hollow body while the second end is in abutment with a flange of an end of the syringe body.

The device of the invention may be used with any standard syringe. It can be placed around the piston rod of a syringe by a user prior to injection, removed from the syringe after injection and, if desired, reused for further injections with different syringes. Further, as the slit in the hollow body is closable, a user will not need to hold the device to the syringe during use as the hollow body fully surrounds the piston rod when the slit is closed.

The hollow body could take various shapes. For example, the body could be cuboid. In one preferred embodiment however, the hollow body is substantially cylindrical. This is advantageous as the rounded surfaces of the cylindrical body will be more comfortable for a user to hold when injecting from a syringe.

The hollow body could be made of a resilient material which allowed the body to be opened and closed about the slit by a user pressing on the sides of the body. In this embodiment, the body could be made in one single piece and would be held closed during injection by the resilience of the material from which it was made.

Preferably however, the device further comprises a hinge, and the body is separated into two portions extending between respective sides of the slit and the hinge. Thus, the body can be opened and closed by rotating one or both of the portions about the hinge.

One of the two portions could be larger than the other. In one preferred embodiment however, each portion comprises one half of the hollow body. This is advantageous in that the syringe can easily be inserted into the body and cradled in one half thereof when the body is open. In one particularly preferred embodiment, each portion of the hollow body comprises a half cylinder.

It is desirable that the device can be kept securely closed in use so that a user does not need to hold the device to the piston rod of the syringe and so that the user can be sure that the device will not work free from the piston rod. The hinge could be biased to hold the two portions in the closed position such that the device is held securely closed around the piston rod in use.
In one preferred embodiment however, the device further comprises means for locking the device closed in use.

The means for locking the device closed in use could for example comprise an adhesive member which is placed across the slit and attached to each of the two portions of the hollow body. In one preferred embodiment however, the locking means comprise first and second inter-engaging locking members provided on the hollow body adjacent the slit on respective sides thereof. Preferably, the means for locking the device closed in use are releasable. This has the advantage that the device will be reusable. More preferably, the first locking member comprises a resilient tab which is adapted to click into engagement in a slot forming the second locking member. In an alternative preferred embodiment, the first locking member comprises a resilient hook which clicks into place over an upstanding member which forms the second locking member. These arrangements are particularly advantageous as they provide a mechanism by which the device can be securely locked shut and which can be opened and closed many times without reducing in effectiveness.

Preferably the hollow body is dimensioned to provide some play between the inside of the hollow body and the piston rod. This has the advantage that the hollow body will not hinder the smooth motion of the piston rod in use.

Preferably, devices of differing lengths can be provided, each different length corresponding to a different dose to be administered from a syringe.

Many individuals who self-inject medication are elderly and/or visually impaired. It is therefore desirable that the users can differentiate as easily as possible between different sizes of device to ensure that they use the correct device for a required dose. Preferably therefore, the devices are colour coded, each size of device being a different colour.

Some users may be severely visually impaired or colour blind such that colour coding is not a suitable means of indicating the size of a device to them. Preferably therefore, devices of different sizes also include tactile indicators of their size. In one particularly preferred embodiment, an indication of the size of a device is written in relief on an outer surface of the device.
Alternatively or additionally, each device may be shaped differently depending on the size of the device in question. In one preferred embodiment, the ends of the device are shaped differently depending on the size of the device.

The device could be made from any suitable material. Preferably however it is made from plastics. Still more preferably, the device is formed by injection moulding. This provides a relatively inexpensive mode of manufacture such that the devices can be manufactured in bulk and can be disposable.

Preferably the device further comprises a visual or tactile indicator of the size of dose or the fraction of a total dose that will be expelled from a syringe when used with the device.

In one preferred embodiment, the device is for use with an autoinjector.

Further the device may be for single use such that the device and the syringe are disposed of after use.

Preferred embodiments of the invention will now be described by way of example only and with reference to the accompanying drawings in which:
Figure 1 is a side elevational view of a syringe including a device according to the invention;
Figure 2A is a cross sectional view of a device according to a first embodiment of the invention when open;
Figure 2B is a cross sectional view of the device of Figure 2A when closed;
Figure 3 is a top perspective view of a device according to a second embodiment of the invention when open;
Figure 4 is an underneath perspective view of the device of Figure 3 when open;
Figures 5A to 5C show devices according to the invention for administering a dose of 20%, 40% and 50% respectively; and
Figure 6 is a perspective view of the device of Figure 5A being placed on a syringe.

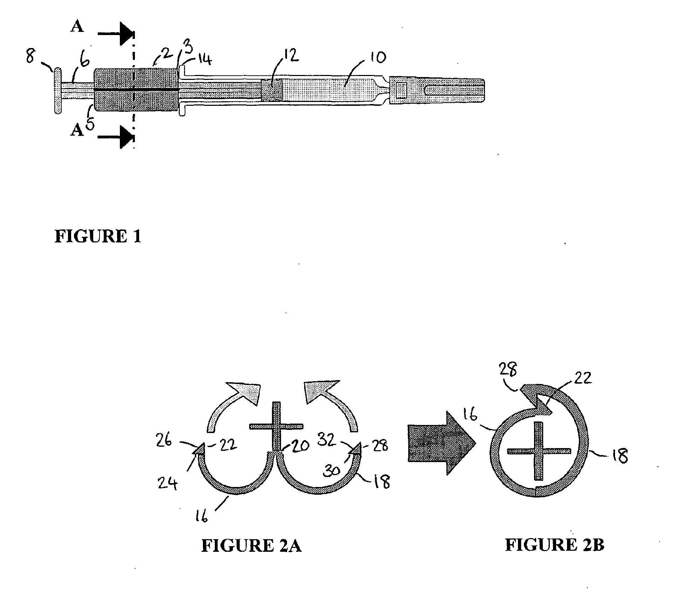

As shown in Figure 1, the invention provides a device 2 which acts as a spacer to limit the dose of a medicament delivered by a syringe 4. A standard syringe of the type with which the device is to be used comprises a piston rod 6 having a flange or finger pad 8 projecting outwardly from the far end thereof. A user pushes the piston rod downwardly to expel fluid from the syringe by pressing on the flange 8 as is known in the art. The main body of the syringe 4 comprises a fluid containing chamber 10, a piston 12 provided at one end of the fluid containing chamber, and a needle provided at the other end of the fluid containing chamber. The piston rod 6 is connected to the piston 12. The end of the syringe body from which the piston rod 6 extends outwardly comprises a radially extending flange 14.

The device 2 is adapted to be received on the syringe 4. As shown in Figures 2A and 2B, the device comprises a hollow body which is substantially cylindrical in form and is made up of a first half 16 and a second half 18, each half being substantially semi circular in cross section. Both the first and second ends of the device are open.

The first and second halves 16, 18 are joined together at respective first edges thereof by a hinge 20 so as to allow the device to be opened and closed as shown by arrows AA. The free second edges of the halves 16, 18 of the device are provided with releasable interlocking click-engagement means which hold the two halves together in the closed position when clicked into position. The interlocking click-engagement means comprise a radially outwardly projecting member 22 provided on the free end of the first half 16 and a second radially inwardly projecting member 28 provided on the free end of the second half 18.

The radially outwardly projecting member 22 is substantially triangular in cross section such that it comprises a first planar surface 24 which extends outwardly perpendicular to the tangent to the cylindrical surface at that point and a second planar surface 26 which extends at an acute angle inwardly and away from the hinge from the radially outer edge of the first planar surface 24 to a point which is in line with the radially inner surface of the first half 16.

The radially inwardly projecting member 28 is also substantially triangular in cross section and is adapted to lockingly engage with the radially outwardly projecting member 22 when the device is closed in use. The radially inwardly projecting member 28 comprises a first planar surface 30 which extends radially inwardly perpendicular to the tangent to the cylindrical surface at that point and a second planar surface 32 which extends at an acute angle outwardly and away from the hinge from the radially inner edge of the first planar surface 30 to a point which is in line with the radially outer surface of the second half 18. When the cylindrical body of the device is closed in use, the radially inwardly projecting member 28 engages over the radially outwardly projecting member 22 such that the first planar surface 30 of the radially inwardly projecting member 28 abuts the first planar surface 24 of the radially outwardly projecting member 22.

In use, a spacer device 2 is provided with a prefilled syringe 4. A user opens the spacer device 2 and places the piston rod 6 of the syringe 4 into one of the halves of the cylindrical body. The user then closes the cylindrical body such that it is locked shut around the piston rod 6 by the click engagement means 22, 28. The position of the device 2 on the piston rod 6 is then adjusted to bring one end 3 of device 2 into abutment with the flange 14 on the end of the fluid chamber 10 of the syringe. Injection of medication from the syringe can then be carried out and injection will stop when the flange 8 at the end of piston rod 6 comes into abutment with the other end 5 of the device 2.

In a preferred embodiment, the inner dimensions of the device 2 are such that there is play between the device and the piston rod 6, once the device is shut around the piston rod 6. In this way the device 2 does not hinder the smooth motion of the piston rod 6. Motion of the piston rod during injection is stopped when the flange 8 at the end of the piston rod comes into abutment with the end 5 of the device 2, and when the end 3 of the device 2 comes into abutment with the flange 14 on the end of the fluid chamber 10 of the syringe.

Once injection has been completed, the device 2 can be removed from the syringe by lifting the radially inwardly projecting member 28 away from radially outwardly projecting member 22 to release the locking means and opening the hollow body by bending the two halves 16, 18 thereof back about the hinge 20. The device can then either be reused with another syringe or can be disposed of.

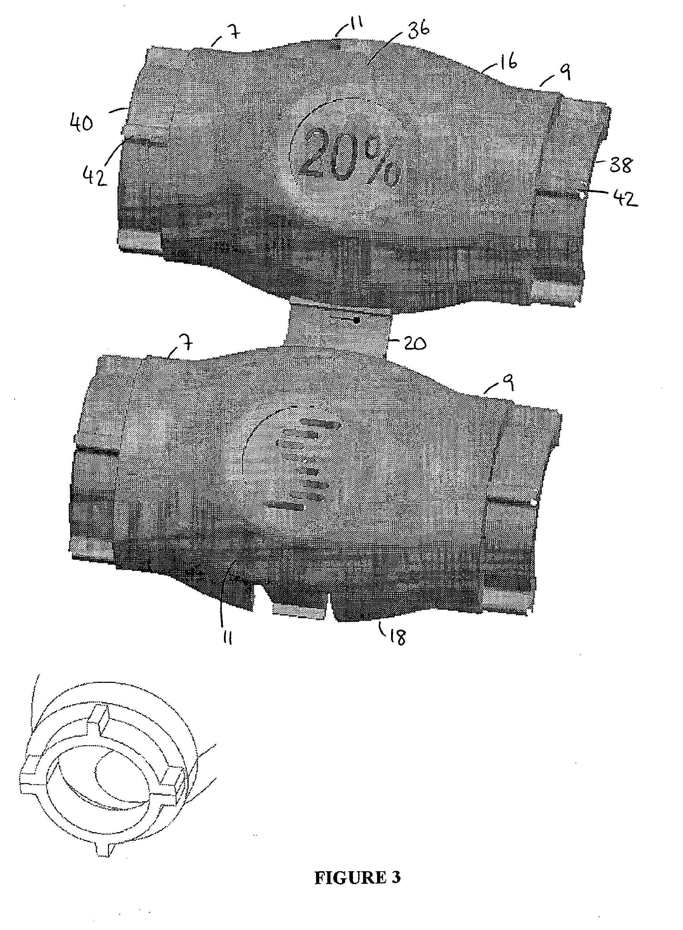

An alternative embodiment of the device 2 is shown in Figure 3. The same reference numbers will be used for equivalent parts. Further, those parts which correspond to those of the first embodiment will not be described again in detail. As shown, the device 2 of the second embodiment is again made up of two halves 16, 18 which are joined together by a hinge 20. The device 2 is also substantially cylindrical in form but the outer surfaces thereof are shaped to be more comfortable for a user to hold. Thus, the diameter of the body 2 increases gradually from longitudinally outer regions thereof toward the centre so that the outer surface of the body curves smoothly radially outwards towards the centre thereof. The diameter of the body then remains constant at the outer ends thereof to provide straight cylindrical portions 7, 9 which are joined to the curved or bulbous central portion 11. As shown in Figure 3, the size of dose obtained with a particular device can be marked on it. In the embodiment shown, the percentage of the maximum dose obtained from a syringe when using the device is given. Thus, for the device of Figure 3, the figure 20% is marked on the widest part 36 of the device. This figure is marked in relief such that it will be recognisable both visually and by touch.

In addition to the above, differently shaped end portions 38, 40 can be provided on the hollow body of device 2. These end portions can be of varying lengths and can include radially projecting ribs 42 as shown. Thus, a visually impaired user can recognise a particular size of device corresponding to a desired dosage by the length of end portions 38, 40 and the existence and number of ribs 42 on them.

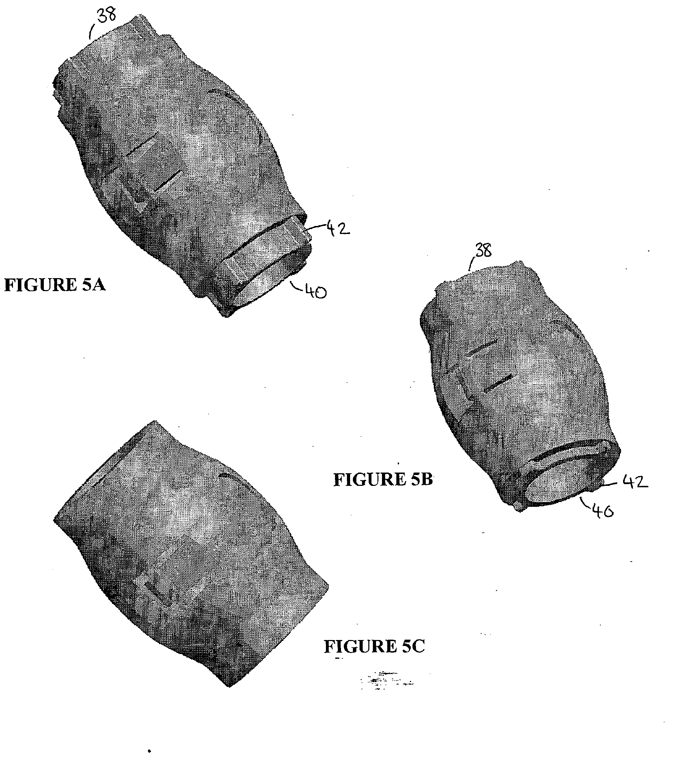

Examples of different end portions 38, 40 for use on devices of different sizes are shown in Figures 5A to 5C. Figure 5A shows the configuration for a dose of 20% corresponding to the Figure 3 embodiment described above. Figure 5B shows the configuration for a dose of 40%. As shown, the end portions 38, 40 are significantly shorter than the end portions of the Figure 5A embodiment. As for the Figure 5A embodiment, four ribs 42 are provided on each of the end portions, equally spaced about the circumference thereof. Figure 5C shows the configuration for a dose of 50%. In this configuration, no end portions at all are provided.

As another means of distinguishing between devices for different dosages, each of the devices of Figures 5A to 5C is preferably a different colour. For example, the 20% dose device of Figure 5A is clear green, the 40% dose device of Figure 5B is burgundy red and the 50% dose device of Figure 5C is dark green.

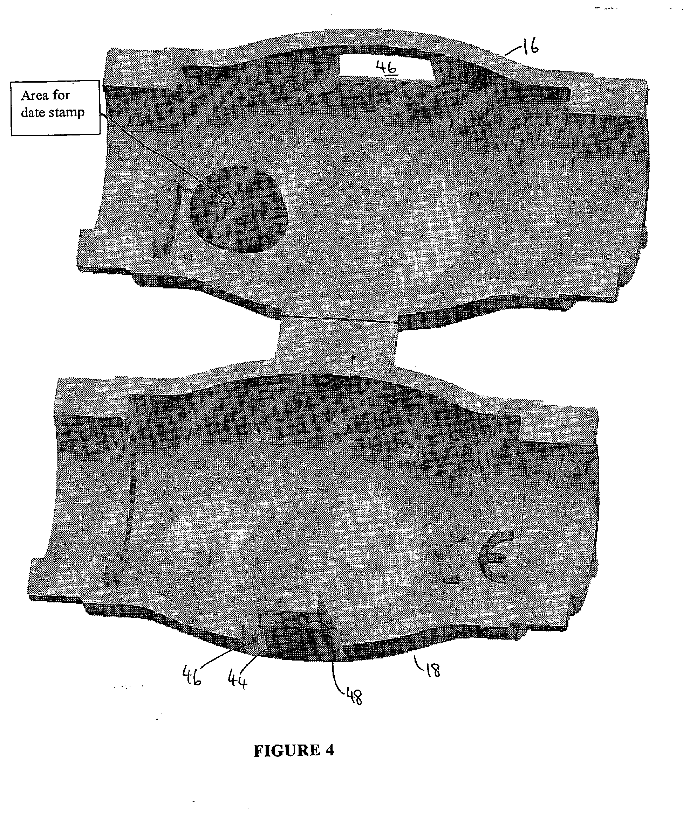

The locking mechanism in the device of Figures 3 and 4 is different from that of the first embodiment and will be described below. It will be appreciated that either of the mechanisms described could be used with any embodiment of the hollow body of device 2. The locking mechanism of the device of Figures 3 and 4 comprises a resilient locking tab 44 provided on second half 18 of the hollow body which is adapted to engage with an aperture 46 provided in first half 16. The aperture 46 is substantially rectangular and is surrounded on all sides by the first half 16. A rectangular portion 46 is cut away from the edge of second half 18 and the locking tab 44 extends from the edge of the cut away portion to a line beyond the free edge of the second half 18. A radially outwardly protruding ridge 48 is provided on the free end of the locking tab 44 as shown.

In use, the first and second halves of the hollow body are brought together such that locking tab 44 is pushed back radially internally of the first half 16. Due to the resilience of the locking tab 44, when the two halves are brought fully together so that the device 2 is closed, the free end of tab 44 springs radially outwardly such that the ridge 48 engages with the aperture 46 thus locking the device in the closed position.

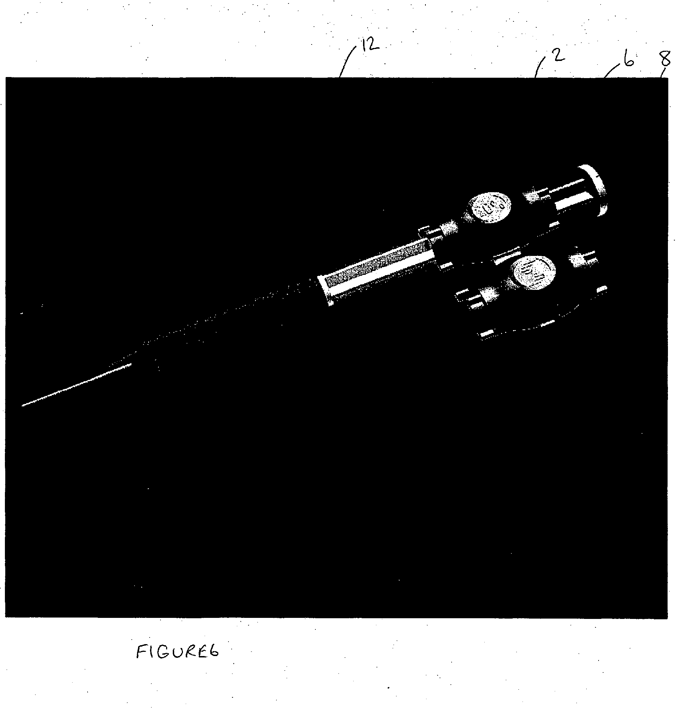

Figure 6 shows the device 2 of Figures 3 and 4 when positioned on a syringe 6 and before being closed as described above. After use, the device can be removed from the syringe by releasing the locking mechanism. To release the locking mechanism, the ridge 48 is pushed radially inwardly through the aperture 46 and the two halves of the hollow body are then opened. Once it has been removed, the device can either be reused with another syringe or it can be disposed of.

The embodiments described above are preferred embodiments only of the invention and are not intended to be limiting. It will be appreciated that various modifications could be made to the embodiments described above which would fall within the scope of the invention as defined in the appended claims.

## Claims

1. A device comprising a syringe (4) and a dose adjusting device (2), the dose adjusting device (2) comprising:
a longitudinal axis;
a hollow body (16, 18) extending about the longitudinal axis;
a first opening provided at a first end (5) of the hollow body (16, 18);
a second opening provided at a second end (3) of the hollow body (16, 18), the second end being opposite to and longitudinally spaced from the first end; and
a slit extending over the length of the hollow body (16, 18),
wherein the hollow body (16, 18) is adapted to be opened about the slit such that in use the hollow body (16, 18) is placed around and closed over the piston rod (6) of the syringe (4),
the device being **characterised in that** the hollow body (16, 18) is movably mountable on the piston rod such that in use, while the second end (3) of the hollow body (16, 18) is already in abutment with a flange (14) of an end of the syringe body, by pushing the piston rod, a flange (8) of the piston rod is brought into abutment with the first end (5) of the hollow body (16, 18).

2. A device as claimed in claim 1, wherein the hollow body (16, 18) is dimensioned to provide some play between the inside of the hollow body and the piston rod.

3. A device as claimed in claim 1 or 2, wherein the hollow body (16, 18) is substantially cylindrical.

4. A device as claimed in any preceding claim, wherein the dose adjusting device (2) further comprises a hinge (20), and the hollow body (16, 18) is separated into two portions extending between respective sides of the slit and the hinge (20).

5. A device as claimed in claim 4, wherein each portion (16,18) comprises a half of the hollow body (16, 18).

6. A device as claimed in any preceding claim, wherein the dose adjusting device (2) further comprises means for locking the dose adjusting device (2) closed in use.

7. A device as claimed in claim 6, wherein the locking means comprise first and second releasably interengaging locking members provided on the hollow body (16, 18) adjacent respective sides of the slit.

8. A device as claimed in claim 6 or 7, wherein the locking means releasably lock the dose adjusting device (2) closed in use.

9. A device as claimed in claim 7 or 8, wherein the first locking member comprises a resilient tab (44) which is adapted to click into engagement in the second locking member, said second locking member comprising a slot (46).

10. A device as claimed in claim 7 or 8, wherein the first locking member comprises a resilient hook (28) which clicks into place over the second locking member, said second locking member comprising an upstanding member (22).

11. A device as claimed in any preceding claim, wherein the dose adjusting device is colour coded, each size of dose adjusting device being a different colour.

12. A device as claimed in any preceding claim, wherein the dose adjusting device further comprises a tactile indicator of its size.

13. A device as claimed in any preceding claim, wherein the dose adjusting device (2) is made from plastics.

14. A device as claimed in any preceding claim, wherein the dose adjusting device (2) further comprises a visual or tactile indicator of the size of dose or the fraction of a total dose that will be expelled from the syringe (4) when used with the dose adjusting device (2).

15. A device as claimed in any preceding claim, wherein the dose adjusting device (2) is for use with an autoinjector.

16. A device as claimed in any preceding claim, wherein the dose adjusting device (2) is for single use such that the syringe and the dose adjusting device (2) are disposed of after use.

## Patentansprüche

1. Vorrichtung, welche eine Spritze (4) und eine Dosierungseinstellungs-Einheit (2) aufweist, wobei die Dosierungseinstellungs-Einheit (2) Folgendes aufweist:
eine Längsachse;
einen Hohlkörper (16, 18), der sich um die Längsachse erstreckt;
eine an einem ersten Ende (5) des Hohlkörpers (16, 18) vorgesehene erste Öffnung;
eine an einem zweiten Ende (3) des Hohlkörpers (16, 18) vorgesehene zweite Öffnung, wobei das zweite Ende dem ersten Ende gegenüber liegt und von diesem in Längsrichtung beabstandet ist; und
einen Schlitz, der sich über die Länge des Hohlkörpers (16, 18) erstreckt,
wobei der Hohlkörper (16, 18) ausgebildet ist, um beim Schlitz geöffnet zu werden, sodass der Hohlkörper (16, 18) bei der Verwendung rund um die Kolbenstange (6) der Spritze (4) angeordnet und über dieser geschlossen werden kann,
**dadurch gekennzeichnet, dass** der Hohlkörper (16, 18) bewegbar auf der Kolbenstange montierbar ist, sodass bei der Verwendung, während das zweite Ende (3) des Hohlkörpers (16, 18) bereits an einem Flansch (14) eines Endes des Spritzenkörpers anliegt, indem auf die Kolbenstange gedrückt wird, ein Flansch (8) der Kolbenstange in eine Lage gebracht wird, in der er an dem ersten Ende (5) des Hohlkörpers (16, 18) anliegt.

2. Vorrichtung nach Anspruch 1, wobei der Hohlkörper (16, 18) so bemessen ist, dass er zwischen der Innenseite des Hohlköpers und der Kolbenstange ein gewisses Spiel bietet.

3. Vorrichtung nach Anspruch 1 oder 2, wobei der Hohlkörper (16, 18) im Wesentlichen zylindrisch ist.

4. Vorrichtung nach einem der vorhergehenden Ansprüche, wobei die Dosierungseinstellungs-Einheit (2) weiters ein Gelenk (20) aufweist, und wobei der Hohlkörper (16, 18) in zwei Teile geteilt ist, die sich zwischen entsprechenden Seiten des Schlitzes und des Gelenks (20) erstrecken.

5. Vorrichtung nach Anspruch 4, wobei jeder Teil (16, 18) eine Hälfte des Hohlkörpers (16, 18) aufweist.

6. Vorrichtung nach einem der vorhergehenden Ansprüche, wobei die Dosierungseinstellungs-Einheit (2) weiters Mittel aufweist, um die Dosierungseinstellungs-Einheit (2) bei der Verwendung in geschlossener Lage zu sperren.

7. Vorrichtung nach Anspruch 6, wobei die Sperrmittel erste und zweite miteinander lösbar in Eingriff gelangende Sperrelemente aufweist, die auf dem Hohlkörper (16, 18) neben entsprechenden Seiten des Schlitzes vorgesehen sind

8. Vorrichtung nach Anspruch 6 oder 7, wobei die Sperrmittel die Dosierungseinstellungs-Einheit (2) bei der Verwendung lösbar in geschlossener Lage sperren.

9. Vorrichtung nach Anspruch 7 oder 8, wobei das erste Sperrelement eine elastische Lasche (44) aufweist, die ausgebildet ist, um in dem zweiten Sperrelement eingreifend einzuschnappen, wobei das zweite Sperrelement einen Schlitz (46) aufweist.

10. Vorrichtung nach Anspruch 7 oder 8, wobei das erste Sperrelement einen elastischen Haken (28) aufweist, der über dem zweiten Sperrelement in einer Stellung einrastet, wobei das zweite Sperrelement ein vorstehendes Element (22) aufweist.

11. Vorrichtung nach einem der vorhergehenden Ansprüche, wobei die Dosierungseinstellungs-Einheit farbkodiert ist, wobei jede Größe einer Dosierungseinstellungs-Einheit eine unterschiedliche Farbe aufweist.

12. Vorrichtung nach einem der vorhergehenden Ansprüche, wobei die Dosierungseinstellungs-Einheit weiters eine taktile Angabe ihrer Größe aufweist.

13. Vorrichtung nach einem der vorhergehenden Ansprüche, wobei, die Dosierungseinstellungs-Einheit (2) aus Kunststoffen hergestellt ist.

14. Vorrichtung nach einem der vorhergehenden Ansprüche, wobei die Dosierungseinstellungs-Einheit (2) weiters eine visuelle oder taktile Angabe der Größe der Dosis oder des Anteils der Gesamtdosis, die von einer Spritze (4) abgegeben werden wird, wenn sie mit der Dosierungseinstellungs-Einheit (2) verwendet wird, aufweist.

15. Vorrichtung nach einem der vorhergehenden Ansprüche, wobei die Dosierungseinstellungs-Einheit (2) zur Verwendung mit einem Autoinjektor vorgesehen ist.

16. Vorrichtung nach einem der vorhergehenden Ansprüche, wobei die Dosierungseinstellungs-Einheit (2) für eine einmalige Verwendung vorgesehen ist, sodass die Spritze und die Dosierungseinstellungs-Einheit (2) nach der Verwendung entsorgt werden.

## Revendications

1. Dispositif comprenant une seringue (4) et un dispositif de réglage de dose (2), le dispositif de réglage de dose (2) comprenant :
un axe longitudinal ;
un corps creux (16,18) s'étendant autour de l'axe longitudinal ;
une première ouverture prévue à une première extrémité (5) du corps creux (16,18) ;
une deuxième ouverture prévue à une deuxième extrémité (3) du corps creux (16, 18), la deuxième extrémité étant opposée à la première extrémité et espacée de celle-ci dans le sens longitudinal ; et
une fente s'étendant sur la longueur du corps creux (16,18) ;
dans lequel le corps creux (16, 18) est adapté pour être ouvert autour de la fente de telle sorte que, lors de l'utilisation, le corps creux (16, 18) est placé autour de la tige de piston (6) de la seringue (4) et fermé sur celle-ci,
le dispositif étant **caractérisé en ce que** le corps creux (16, 18) peut être monté de façon mobile sur la tige de piston de sorte que, lors de l'utilisation, alors que la deuxième extrémité (3) du corps creux (16, 18) est déjà en appui contre une collerette (14) d'une extrémité du corps de seringue, en poussant la tige de piston, une collerette (8) de la tige de piston est mise en appui contre la première extrémité (5) du corps creux (16, 18).

2. Dispositif selon la revendication 1, dans lequel le corps creux (16, 18) est dimensionné pour présenter un certain jeu entre l'intérieur du corps creux et la tige de piston.

3. Dispositif selon la revendication 1 ou 2, dans lequel le corps creux (16, 18) est sensiblement cylindrique.

4. Dispositif selon l'une quelconque des précédentes revendications, dans lequel le dispositif de réglage de dose (2) comprend en outre une charnière (20), et le corps creux (16, 18) est séparé en deux parties qui s'étendent entre des côtés respectifs de la fente et de la charnière (20).

5. Dispositif selon la revendication 4, dans lequel chaque partie (16, 18) constitue une moitié du corps creux (16, 18).

6. Dispositif selon l'une quelconque des précédentes revendications, dans lequel le dispositif de réglage de dose (2) comprend en outre un moyen pour bloquer le dispositif de réglage de dose (2) en position fermée lors de l'utilisation.

7. Dispositif selon la revendication 6, dans lequel le moyen de blocage comprend des premier et deuxième éléments de blocage s'accrochant mutuellement de manière détachable prévus sur le corps creux (16, 18) de façon adjacente aux côtés respectifs de la fente.

8. Dispositif selon la revendication 6 ou 7, dans lequel le moyen de blocage bloque de façon détachable le dispositif de réglage de dose (2) en position fermée lors de l'utilisation.

9. Dispositif selon la revendication 7 ou 8, dans lequel le premier élément de blocage comprend une patte élastique (44) qui est adaptée pour s'enclencher en venant en contact avec le deuxième élément de blocage, ledit deuxième élément de blocage comprenant une fente (46).

10. Dispositif selon la revendication 7 ou 8, dans lequel le premier élément de blocage comprend un crochet élastique (28) qui s'enclenche en place sur le deuxième élément de blocage, ledit deuxième élément de blocage comprenant un élément vertical (22).

11. Dispositif selon l'une quelconque des précédentes revendications, dans lequel le dispositif de réglage de dose est codé par couleur, chaque taille de dispositif de réglage de dose étant d'une couleur différente.

12. Dispositif selon l'une quelconque des précédentes revendications, dans lequel le dispositif de réglage de dose comprend en outre un indicateur tactile qui indique sa taille.

13. Dispositif selon l'une quelconque des précédentes revendications, dans lequel le dispositif de réglage de dose (2) est en matière plastique.

14. Dispositif selon l'une quelconque des précédentes revendications, dans lequel le dispositif de réglage de dose (2) comprend en outre un indicateur visuel ou tactile représentant le volume de dose ou la fraction d'une dose totale qui sera expulsée de la seringue (4) lorsqu'elle est utilisée avec le dispositif de réglage de dose (2).

15. Dispositif selon l'une quelconque des précédentes revendications, dans lequel le dispositif de réglage de dose (2) est prévu pour être utilisé avec un auto-injecteur.

16. Dispositif selon l'une quelconque des précédentes revendications, dans lequel le dispositif de réglage de dose (2) est prévu pour un usage unique de sorte que la seringue et le dispositif de réglage de dose (2) sont jetés après l'usage.
